# EUROPEAN PATENT APPLICATION

(11) **EP 3 187 193 A1**
(43) Date of publication of application: **05.07.2017**
(21) Application number: 15834896.1
(22) Date of filing: 03.08.2015
(51) Int. Cl.: A61L 2/10, A61C 19/02, A61C 19/04

(54) **AUTOMATIC ORAL SENSOR STERILIZING AND PACKAGING SYSTEM**

(30) Priority: 28.08.2014 KR 20140112890; 06.03.2015 KR 20150031602
(71) Applicant: Rayence Co., Ltd., Hwaseong-si, Gyeonggi-do 445-170 (KR); Vatech Ewoo Holdings Co., Ltd, Gyeonggi-do 445-170 (KR)
(72) Inventor: YUN, Min-seok, Hwaseong-si Gyeonggi-do 445-170 (KR); KIM, Dong Soo, Hwaseong-si Gyeonggi-do 445-170 (KR); KIM, Tae Woo, Hwaseong-si Gyeonggi-do 445-170 (KR)
(74) Representative: Markfort, Iris-Anne Lucie
(86) International application number: PCT/KR2015/008091
(87) International publication number: WO 2016/032141

(57) **Abstract**

The present invention relates to an automatic oral sensor sterilizing and packaging system and, particularly, to an automatic oral sensor sterilizing and packaging system capable of automatically sterilizing an oral sensor and further packaging the oral sensor with disposable wrapping paper, comprising: a housing including a sterilization unit providing a sterilization region for sterilization of the oral sensor and/or a packaging unit providing a packaging region for packaging the oral sensor; and a transfer unit for making the oral sensor pass through at least one of the sterilization region and the packaging region.

## Description

### Technical Field

The present invention relates generally to an automatic oral sensor sterilizing and packaging system. More particularly, the present invention relates to an automatic oral sensor sterilizing and packaging system, which is configured to automatically sterilize an oral sensor and package the same with disposable wrapping paper, whereby it is possible to prevent cross infection caused by bacteria and blood when a structure inside a subject's mouth is radiographed by using the oral sensor.

### Background Art

Generally, in dental clinics, to radiograph a structure inside a subject's mouth, an oral sensor is directly inserted inside the subject's mouth, and force is exerted on the oral sensor whereby the sensor makes contact with the mouth. Due to this, a perforation may occur in wrapping paper wrapping the oral sensor, and the oral sensor may come into contact with saliva or blood, whereby contamination may occur.

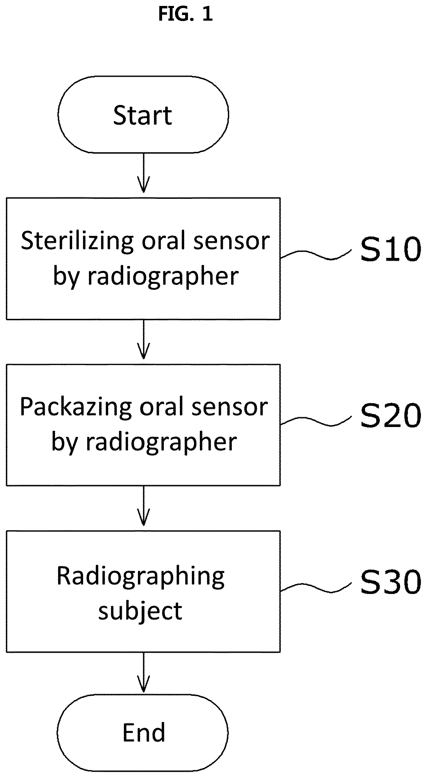

By the above described contamination of the oral sensor, cross infection between subjects may occur. To prevent such cross infection, before radiography, as shown in FIG. 1, a radiographer disinfects the oral sensor with alcohol (S10), then packages the oral sensor with disposable wrapping paper made of a sanitary plastic material (S20), and a structure inside a subject's mouth is radiographed to obtain information thereof (S30).

However, the above described conventional technology is problematic in that since the radiographer may neglect the oral sensor after radiography, and if the oral sensor is left unattended exposed to various bacteria and impurities, it becomes unhygienic. The conventional technology is further problematic in that since the radiographer his/herself disinfects the oral sensor with alcohol, it is difficult to identify whether sterilization is completed or not, and after sterilization, in the process of packaging, the oral sensor may be recontaminated, whereby cross infection between subjects may occur. As a consequence, dental patients may have anxiety concerning oral radiography.

Further, the conventional technology is further problematic in that since the radiographer his/herself disinfects and packages the oral sensor, the process of disinfection and packaging is inconvenient, and a radiographer's medical efficiency may be lowered because the process of disinfection and packaging should be repeated whenever performing radiography.

### Disclosure

### Technical Problem

Accordingly, the present invention has been made keeping in mind the above problems occurring in the related art, and the present invention is intended to propose an automatic oral sensor sterilizing and packaging system, which is configured to automatically sterilize an oral sensor and package the same with disposable wrapping paper, whereby it is possible to prevent cross infection caused by bacteria and blood when a structure inside a subject's mouth is radiographed by using the oral sensor, and it is possible to improve a radiographer's medical efficiency.

### Technical Solution

In order to achieve the above object, according to one aspect of the present invention, there is provided an automatic oral sensor sterilizing and packaging system including: an oral sensor loader configured to provide a seat allowing an oral sensor to be loaded thereon, and to generate a loading signal when the oral sensor is loaded on the seat; a sterilizer configured to sterilize the oral sensor as the seat enters a sterilization zone; a packaging unit configured to package the oral sensor with a wrapping paper as the seat enters a packaging zone; a mover configured to transfer the seat to the sterilization zone in response to input of a first transfer signal, and transfer the seat to the packaging zone in response to input of a second transfer signal; and a controller configured to generate the first transfer signal in response to input of the loading signal, and generate the second transfer signal as the sterilizer completes sterilization.

The automatic oral sensor sterilizing and packaging system may further include: a housing configured to accommodate the oral sensor loader, the sterilizer, the packaging unit, the mover, and the controller therein; and a door configured to expose the seat to an outside by being openably provided at a side of the housing.

Meanwhile, the automatic oral sensor sterilizing and packaging system may further include: a motion recognition sensor provided at a side of the housing, and configured to generate a motion recognition signal in response to recognition of motion of the housing, wherein the door is opened in response to input of the motion recognition signal.

The automatic oral sensor sterilizing and packaging system may further include a bed provided at a lower portion of the seat and configured to cover an inside of the housing when the door is opened.

Herein, the seat may include: a first part configured to allow a sensor part of the oral sensor to be loaded thereon; a second part configured to allow a cable of the oral sensor to be loaded thereon; and an anti-twist part provided in the second part, and configured to guide the cable while preventing the cable from being twisted.

Further, the sterilizer may include: a light source configured to emit light for sterilization inside the sterilization zone; and a reflector configured to reflect the light for sterilization inside the sterilization zone.

Here, the wrapping paper may be in an envelope shape with a mouth thereof being open, and the packaging unit may include a wrapping paper stacker configured to allow the wrapping paper to be stacked therein and an opener configured to open the mouth of a wrapping paper in the packaging zone, of stacked wrapping paper.

Meanwhile, the opener may be provided with a sucker configured to suck a surface of the wrapping paper and move up and down, such that the sucker is moved down to suck the surface of the wrapping paper, and then moves up to open the mouth.

Further, the mover may be configured to move the seat vertically and horizontally, and the sterilizer and the packaging unit may be disposed on top of the other at a side of a vertical path of the seat.

In order to achieve the above object, according to another aspect of the present invention, there is provided an automatic oral sensor sterilizing and packaging system including: a housing provided with at least one of a sterilizer providing a sterilization zone to sterilize an oral sensor, and a packaging unit providing a packaging zone to package the oral sensor; and a mover configured to pass the oral sensor through at least one of the sterilization zone and the packaging zone.

Here, the sterilizer may be configured to either emit light for sterilization or spray sterilizing liquid to the sterilization zone.

Further, the sterilizer may include: a light source configured to emit light for sterilization to the sterilization zone; and a reflector configured to reflect the light for sterilization to the sterilization zone.

Meanwhile, the packaging unit may include: a wrapping paper supplier configured to supply wrapping paper that is in an envelope shape with a mouth thereof being open, to the packaging zone; and an opener configured to open the mouth of the wrapping paper.

Further, the opener may include: a sucker configured to suck an outer surface of the wrapping paper, and to be movable in an opening direction of the mouth, wherein the sucker opens the mouth by sucking the outer surface of the wrapping paper and by moving in the opening direction.

Meanwhile, the mover may include a seat allowing the oral sensor to be loaded thereon, and may be configured to pass the seat through at least one of the sterilization zone and the packaging zone.

Here, the sterilization zone and the packaging zone may be disposed along a first direction, and the mover may be configured to move the seat in the first direction, and in a second direction intersecting with the first direction.

The automatic oral sensor sterilizing and packaging system may further include a door openably provided at a side of the housing, such that the oral sensor is loaded on the seat through the door.

Meanwhile, the automatic oral sensor sterilizing and packaging system may further include a motion recognition sensor provided at a side of the housing, and configured to open and close the door in response to a user's motion.

### Advantageous Effects

According to the present invention having the above-described characteristics, it is possible to prevent cross infection caused by bacteria and blood when a structure inside a subject's mouth is radiographed by using an oral sensor by providing function to automatically sterilize the oral sensor and package the same, thereby contributing to subject's health, and also it is possible to improve a radiographer's medical efficiency by performing actions of the radiographer instead.

### Description of Drawings

FIG. 1 is a view showing a process of sterilization and packaging of a conventional oral sensor;
FIG. 2 is a view showing an automatic oral sensor sterilizing and packaging system according to an embodiment of the present invention;
FIGS. 3A to 3B are views showing an oral sensor loader of the automatic oral sensor sterilizing and packaging system according to an embodiment of the present invention;
FIGS. 4A to 4D are views showing a sterilizer of the automatic oral sensor sterilizing and packaging system according to an embodiment of the present invention;
FIGS. 5A to 5C are views showing a packaging unit of the automatic oral sensor sterilizing and packaging system according to an embodiment of the present invention;
FIG. 6 is a flow chart showing operation of the automatic oral sensor sterilizing and packaging system according to an embodiment of the present invention; and
FIGS. 7A to 7G are views showing operation of the automatic oral sensor sterilizing and packaging system according to an embodiment of the present invention.

**[Description of reference numerals] [34]**

| | |
|---|---|
| 100: oral sensor loader | 110: door |
| 120: seat | 130: bed |
| 200: sterilizer | 210: light source |
| 220: reflector | 300: packaging unit |
| 310: wrapping paper stacker | 311: alignment guide |
| 312: elastic member | 313: rotational driving member |
| 320: opener | 321: sucker |
| 400: mover | 500: controller |
| 600: motion recognition sensor | 610: door button |
| 620: display unit | 700: cover |
| 1000: oral sensor | 2000∼2002: wrapping paper |

### Mode for Invention

Hereinbelow, exemplary embodiments of the present invention will be described in detail with reference to the accompanying drawings. The present invention may, however, be embodied in many different forms and should not be construed as being limited to only the embodiments set forth herein. Also, for convenience of understanding of the elements, in the figures, sizes or thicknesses may be exaggerated to be large (or thick), may be expressed to be small (or thin) or may be simplified for clarity of illustration, but due to this, the protective scope of the present invention should not be interpreted narrowly. Throughout the drawings, the same reference numerals will refer to the same or like parts.

It will be understood that when an element is referred to as being "coupled" or "connected" to another element, it can be directly coupled or connected to the other element or intervening elements may be present therebetween. It will be further understood that the terms "comprises", "comprising", "includes", and/or "including" when used herein, specify the presence of stated features, integers, steps, operations, elements, and/or components, but do not preclude the presence or addition of one or more other features, integers, steps, operations, elements, components, and/or groups thereof.

It will be understood that, although the terms first, second, etc. may be used herein to describe various elements, these elements should not be limited by these terms. For instance, a first element discussed below could be termed a second element without departing from the teachings of the present invention. Similarly, the second element could also be termed the first element.

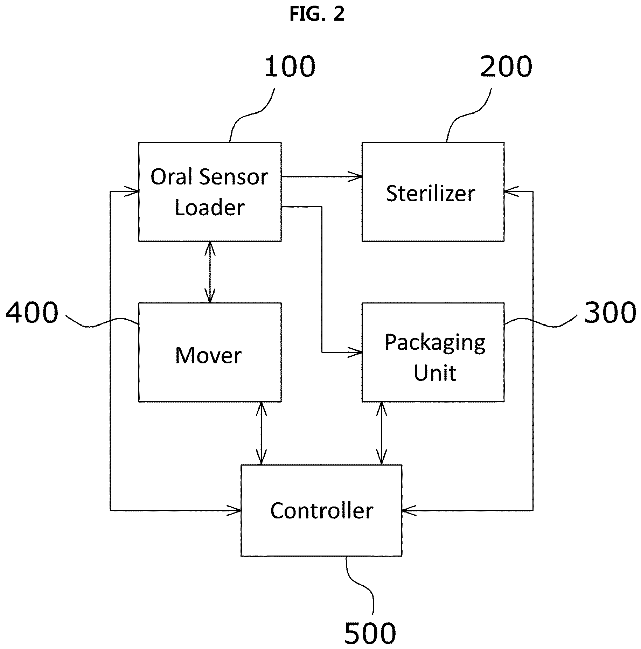

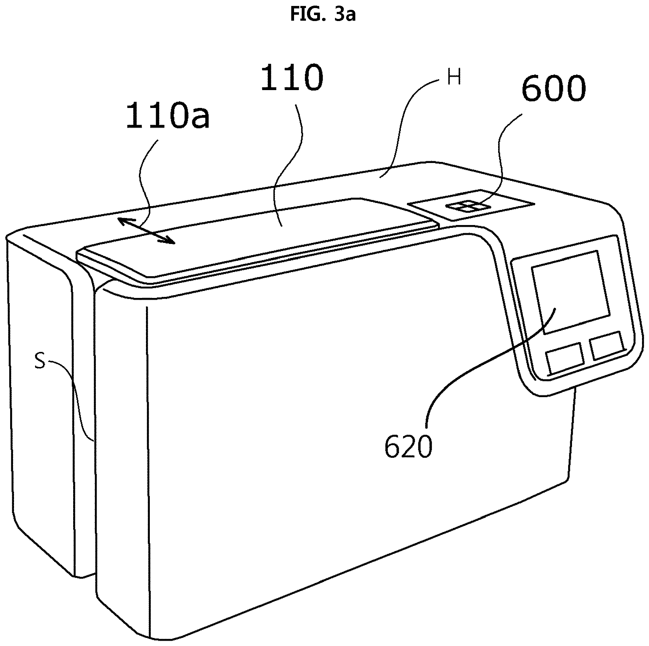

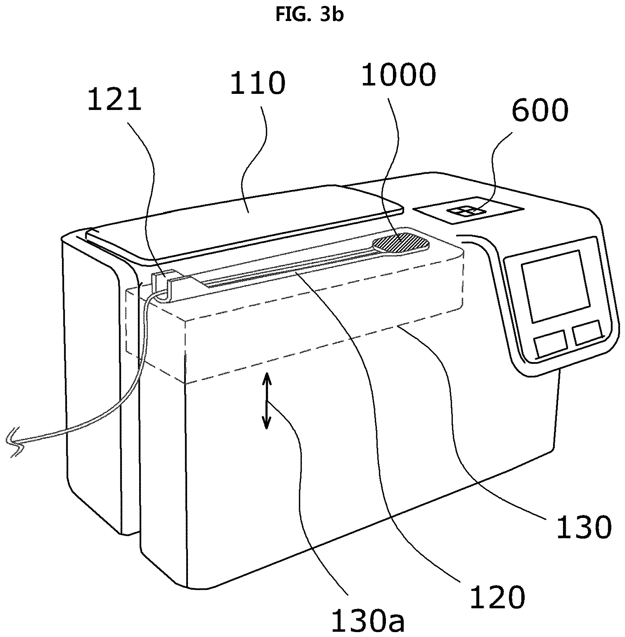

FIG. 2 is a view showing an automatic oral sensor sterilizing and packaging system according to an embodiment of the present invention. An automatic oral sensor sterilizing and packaging system according to an embodiment of the present invention includes: an oral sensor loader 100; a sterilizer 200; a packaging unit 300; a mover 400; and a controller 500. Here, FIGS. 3A to 3B are views showing the oral sensor loader 100 of the automatic oral sensor sterilizing and packaging system according to an embodiment of the present invention, and referring to FIGS. 2 to 3B, a detailed description of the automatic oral sensor sterilizing and packaging system according to the embodiment of the present invention is as follows.

The oral sensor loader 100 is configured to provide a seat 120 allowing an oral sensor 1000 to be loaded thereon, to generate a loading signal when the oral sensor 1000 is loaded on the seat, and to output the generated loading signal to the controller 500. Herein, the oral sensor loader 100 may include a sensor (not shown) to determine whether the oral sensor 1000 is desirably loaded on the seat 120, wherein the sensor may be a position sensor or a light sensor, but is not limited thereto. Meanwhile, the oral sensor loader 100 may include a door 110, the seat 120, and a bed 130.

Herein, the door 110 is configured to be opened so that a system user can load the oral sensor 1000 on the seat 120, and to be closed when the seat 120 is moved down by a mover 400 after the system user the oral sensor 1000 on the seat 120. Here, as shown in FIG. 3A, it is preferred that the door 110 is a sliding door that is opened and closed in a horizontal direction 110a by the mover 400 having received a first transfer signal of the controller 500, but it may be a rotary door using a hinge. Further, the door 110 may be automatically opened and closed by a motor (not shown) of the mover 400, or may be manually opened and closed. Particularly, for system user convenience and hygiene, a motion recognition sensor 600 may be provided outside the system, and the system user may open and close the door 110 without touching the system when a user places his/her palm near the motion recognition sensor 600. Meanwhile, instead of the motion recognition sensor 600, a device where UX (user experience) technology is applied, such as a voice recognition sensor (not shown), may be used to open and close the door 110 without touching the system.

Further, the seat 120 allows the oral sensor 1000 to be loaded at a desired location without special attention of the system user, and may include: a first part configured to allow a sensor part of the oral sensor 1000 thereon; a second part configured to allow a cable of the oral sensor 1000 to be loaded thereon; an anti-twist part 121 provided in the second part, and configured to prevent the cable from being twisted when the seat 120 is moved. Herein, the anti-twist part 121 may be in a hook shape or in a shape similar to a hook to allow the cable of the oral sensor 1000 to be moved forward and backward and to prevent the same from being moved left and right by generously surrounding the cable. Meanwhile, a slit or a gap S allowing the cable to pass therethrough in the process of transferring the oral sensor 1000 for sterilization and packaging may be provided at a side of a housing H along a longitudinal direction thereof in the automatic oral sensor sterilizing and packaging system according to the present invention.

Meanwhile, if the oral sensor 1000 is a wireless type, the seat 120 may be not provided with the second part nor the anti-twist part 121.

Meanwhile, the bed 130 is provided at a lower portion of the seat 120 and is configured to cover the inside of the system so that the system user cannot see the sterilizer 200, and the like, when the door 110 is opened. Herein, the bed 130 is connected to an end of the seat 120 to be spaced apart therefrom at a predetermined interval, and may be configured to be moved in a vertical direction 130a as the mover 400 moves the seat 120, or the mover 400 may move the seat 120 in a horizontal direction separately from the bed 130.

Further, the sterilizer 200 is configured to sterilize the oral sensor loaded on the seat 120 as the seat 120 of the oral sensor loader 100 enters a sterilization zone, and to complete sterilization in response to control of the controller 500 after a predetermined sterilization time. Herein, it is preferred that the sterilizer 200 applies a dry sterilization method using an ultraviolet lamp, a cold cathode tube, or the like, but not limited thereto, the sterilizer 200 may apply a wet sterilization method that sprays sterilizing liquid, such as alcohol.

Meanwhile, the packaging unit 300 is configured to package the oral sensor loaded on the seat 120 with a wrapping paper as the seat 120 of the oral sensor loader 100 enters a packaging zone. Herein, it is preferred that the wrapping paper supplied to the packaging unit 300 is disposable double wrapping paper, but is not limited thereto.

Here, the sterilization zone of the sterilizer 200 and the packaging zone of the packaging unit 300 may be arranged in a first direction, for example, a vertical direction, wherein the vertical position can be switched to each other.

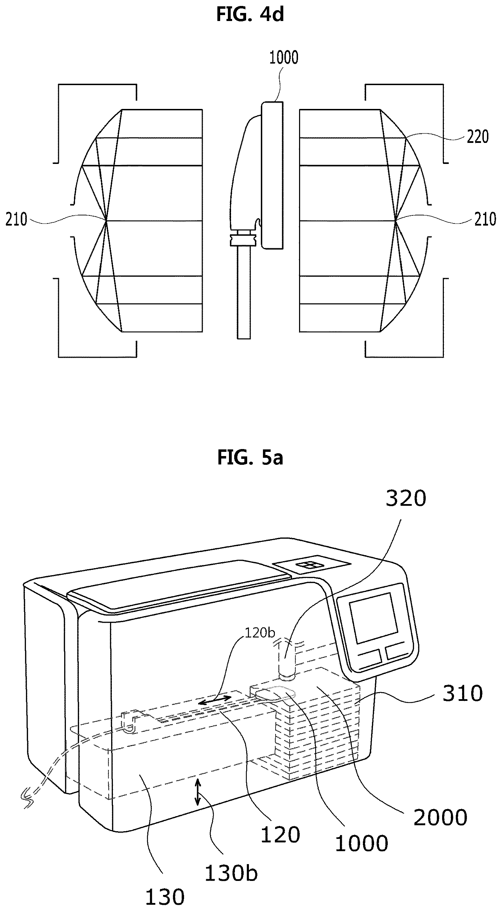

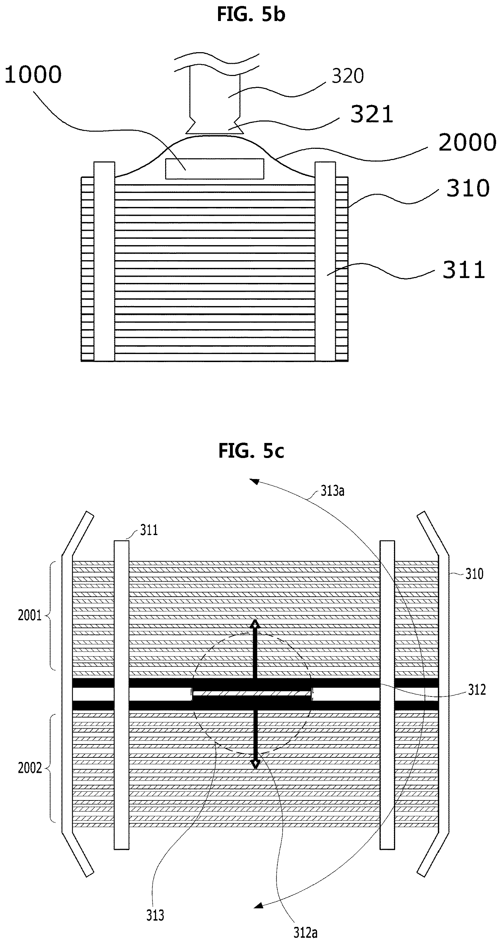

Further, the mover 400 is configured to transfer the seat 120 of the oral sensor loader 100 to the sterilization 200 in response to input of a first transfer signal from the controller 500, and transfer the seat 120 of the oral sensor loader 100 to the packaging unit 300 in response to input of a second transfer signal from the controller 500. Herein, the mover 400 is configured to move the seat 120 in the first direction, and in a second direction intersecting with the first direction, for example, a vertical direction and a horizontal direction, in response to the control of the controller 500. In other words, the mover 400 is a means for moving all the components required to be moved in the system (for example, the door 110, the seat 120 and the bed 130, and an opener 320 and a rotational driving member 313 shown in FIGS. 5A to 5C, which will be described hereinafter) in response to the control of the controller 500, and may include: an up/down driver and motor for moving in a vertical direction; and a conveying belt and a linear motor for moving in a horizontal direction, which will be easily understood by those skilled in the art, so the detailed connection therebetween is not shown in the accompanying drawings for the sake of convenience.

Meanwhile, the controller 500 is configured to generate the first transfer signal in response to input of the loading signal from the oral sensor loader 100, and output the generated first transfer signal to the mover 400, and generate the second transfer signal in response to input of a sterilization signal from the sterilizer 200 and output the generated second transfer signal to the mover 400. Herein, the controller 500 may be configured to control power to be supplied to the sterilizer 200 when the loading signal is input from the oral sensor loader 100, thereby reducing power consumption, but is not limited thereto.

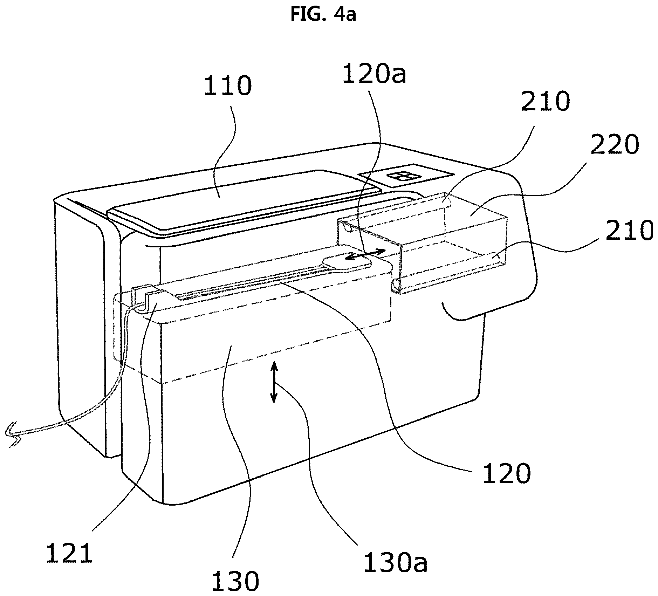

FIGS. 4A to 4D are views showing the sterilizer 200 of the automatic oral sensor sterilizing and packaging system according to an embodiment of the present invention, and the sterilizer 200 according to the embodiment of the present invention includes a light source 210 and a reflector 220.

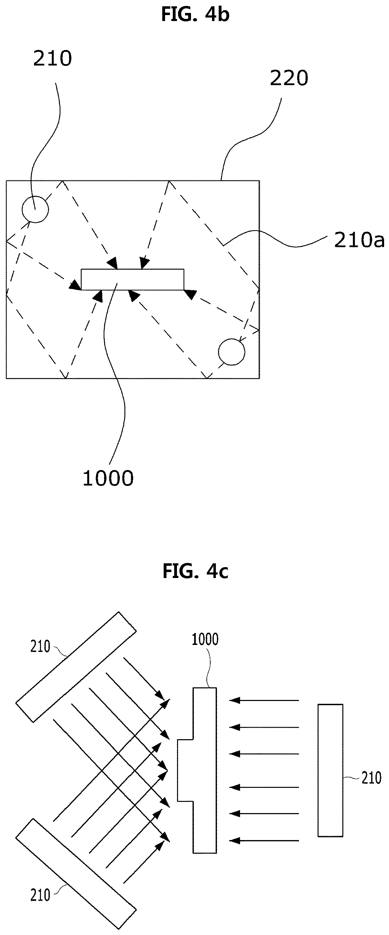

The light source 210 is configured to emit light for sterilization 210a as the seat 120 of the oral sensor loader 100 enters the sterilization zone. Herein, the light source 210, as shown in FIGS. 4A to 4B, may be two ultraviolet lamps provided at upper left and lower right ends of the oral sensor 1000 respectively, but the number of the ultraviolet lamps may be increased or decreased, the location of the ultraviolet lamp may be changed to, for example, an upper right end, or a lower left end. Here, it is preferred that the ultraviolet lamp is a lamp for emitting UV-C having a wavelength range of 100 to 280nm, in particular, a wavelength of about 253nm, thereby improving sterilization efficiency, but a cold cathode tube may be used for miniaturization. Further, as shown in FIG. 4C, the light source 210 may be disposed at three different directions based on the oral sensor 1000 to effectively sterilize an uneven portion of the oral sensor 1000.

Meanwhile, the light source 210 may be configured to be activated by being supplied with power by the control of the controller 500 when the mover 400 moves the seat 120 to a location where the seat can enter the sterilization zone of the sterilizer 200 (moving in the vertical direction 130a), thereby emitting the light for sterilization 210a, or may be configured to be activated by being supplied with power by the control of the controller 500 as the seat 120 enters the sterilization zone (moving in the horizontal direction 120a), thereby emitting the light for sterilization 210a, whereby it is possible to reduce power consumption, but is not limited thereto.

Further, the reflector 220 is made of a material with high reflectance, such as aluminum, and is configured to reflect the light for sterilization 210a emitted from the light source 210 within the sterilization zone, and provide the reflected light for sterilization 210a to the oral sensor 1000. In other words, when the oral sensor 1000 loaded on the seat 120 is moved in the vertical direction 130a by the mover 400, and then is moved in the horizontal direction 120a to enter the sterilization zone defined as the inside of the sterilizer 200, as shown in FIG. 4B, the reflector 220 reflects the light for sterilization 210a emitted from the light source 210 such that the oral sensor 1000 is disinfected. Meanwhile, as shown in FIG. 4D, the reflector 220 may be in a lamp shade shape.

Here, in general, a separation distance between the oral sensor 1000 and the light source 210 is determined within a range between 10 and 15cm, and after the light for sterilization 210a of the light source 210 is emitted to the oral sensor 1000, three to five minutes later, the sterilization process is completed. However, it is possible to reduce sterilization time by reducing the separation distance, and it is possible to increase or reduce sterilization time in consideration of desired sterilization power (*µ*W×sec/cm²).

FIGS. 5A to 5C are views showing the packaging unit 300 of the automatic oral sensor sterilizing and packaging system according to an embodiment of the present invention, and the packaging unit 300 according to the embodiment of the present invention includes: a wrapping paper stacker 310; and an opener 320.

The wrapping paper stacker 310, which is a kind of a wrapping paper supplier, is configured to allow wrapping paper 2000 for packaging the oral sensor 1000 to be stacked therein, and to supply the stacked wrapping paper 2000 to the packaging zone, wherein the wrapping paper stacker may be provided with an alignment guide 311 for aligning the wrapping paper 2000. Herein, the wrapping paper 2000 may be in an envelope shape with a side thereof facing the seat 120 being open or in a shape similar thereto. Further, in order to realize an automatic packaging function, the mover 400 moves the seat 120 with the oral sensor 1000 loaded thereon to a location corresponding to a stack height of the wrapping paper 2000 stacked in the wrapping paper stacker 310. In order to match the seat 120 with the stack height, the controller 500 may control the mover 400 by using a position sensor (not shown), or may control the mover 400 in response to a predetermined stack height by using an elastic member (not shown) provided at a lower portion of the wrapping paper stacker 310 to keep the stack height constant regardless of the amount of the wrapping paper 2000 stacked in the wrapping paper stacker 310.

Here, the wrapping paper stacker 310 may further include a rotational driving member 313 that can rotate the wrapping paper stacker 310, 180 degrees 313a, to supply two different kinds, for example, different sizes, of wrapping paper 2001 and 2002. Herein, the wrapping paper stacker 310 is configured to be provided with an elastic member 312 on an interface between the two kinds of wrapping paper 2001 and 2002, wherein the elastic member 312 can exert force in an outer direction 312a such that each of the wrapping paper 2001 and 2002 has a predetermined stack height, but is not limited thereto.

Further, the opener 320 is provided with a sucker 321 at an end thereof, and is configured to open a mouth of the wrapping paper 2000 by sucking a surface of the wrapping paper 2000 with the sucker 321. Here, the controller 500 may be configured to move down the sucker 321 of the opener 320 to suck the wrapping paper as the seat 120 with the oral sensor 1000 loaded thereon enters the packaging zone by being moved in the horizontal direction 120b after being moved in the vertical direction 130b by the mover 400, and then move up the sucker 321 of the opener 320 to open the mouth of the wrapping paper 2000, but not limited thereto, and the opener 320 may be configured to open the mouth of uppermost wrapping paper 2000 in advance regardless of the seat 120 with the oral sensor 1000 loaded thereon entering the packaging zone.

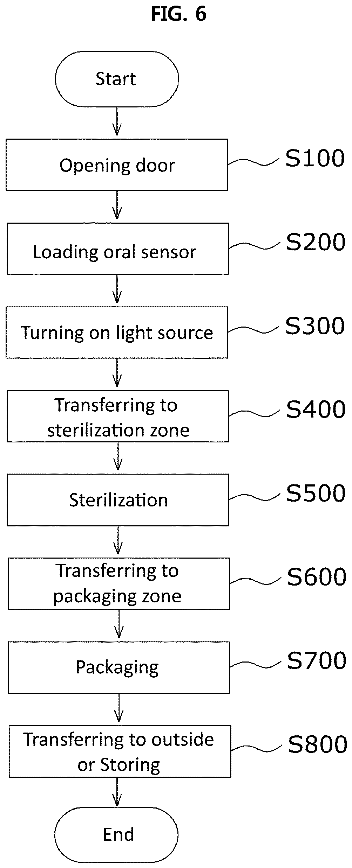

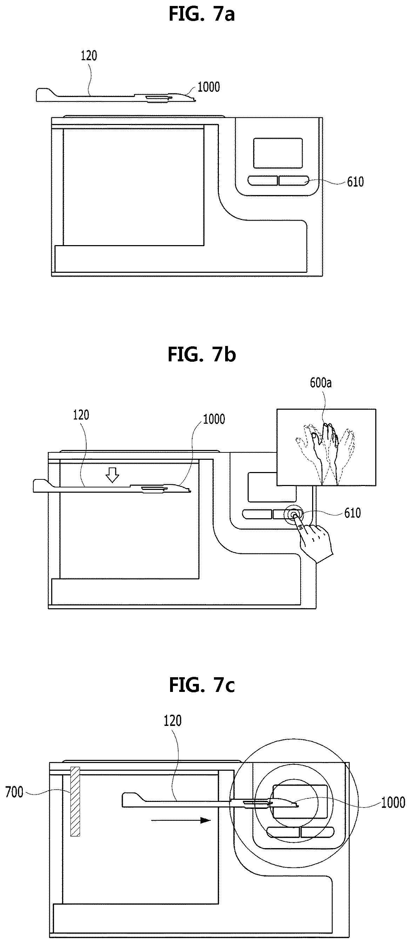

FIG. 6 is a flow chart showing operation of the automatic oral sensor sterilizing and packaging system according to an embodiment of the present invention; and FIGS. 7A to 7G are views showing operation of the automatic oral sensor sterilizing and packaging system according to an embodiment of the present invention. Referring to FIGS. 1 to 7G, detailed description of operation of the automatic oral sensor sterilizing and packaging system according to the embodiment of the present invention is as follows.

Firstly, a system user opens the door 110 by placing his/her palm near the motion recognition sensor 600 provided outside the system (S100). Of course, instead of the motion recognition sensor 600, the door 110 may be opened by using a device where UX (user experience) technology is applied, such as a voice recognition sensor (not shown), or by using a door button 610 provided outside the system.

Then, as shown in FIG. 7A, the system user loads the oral sensor 1000 on the seat 120 (S200). Here, when the oral sensor 1000 is loaded on the seat 120, as shown in FIG. 7B, the seat 120 is moved down by the mover 400 while the door 110 is closed. Herein, it is preferred that the door 110 is configured to be automatically closed by sensing load of the oral sensor 1000, but the door may be configured to be closed by a movement of a hand near the motion recognition sensor 600 600a or by using the door button 610.

Then, as shown in FIG. 7B, when the seat 120 with the oral sensor 1000 loaded thereon is moved down to a height where the seat can enter the sterilization zone, the controller 500 turns on the light source 210 by supplying power thereto (S300).

Then, as shown in FIG. 7C, the seat 120 with the oral sensor 1000 loaded thereon enters the sterilization zone by being moved horizontally by the mover 400 (S400). Here, in order to prevent the light for sterilization 210a from being emitted outside the system, it is preferred that a cover 700 that is a shutter for covering the gap S of the housing H is closed.

Next, the light source 210 emits the light for sterilization 210a as the seat 120 with the oral sensor 1000 loaded thereon enters the sterilization zone, thereby performing sterilization operation (S500). Herein, in general, the separation distance between the oral sensor 1000 and the light source 210 is determined within a range between 10 and 15cm, and after the light for sterilization 210a of the light source 210 is emitted to the oral sensor 1000, three to five minutes later, the sterilization process is completed. However, it is possible to reduce sterilization time by reducing the separation distance, and it is possible to increase or reduce sterilization time in consideration of desired sterilization power (*µ*W×sec/cm²).

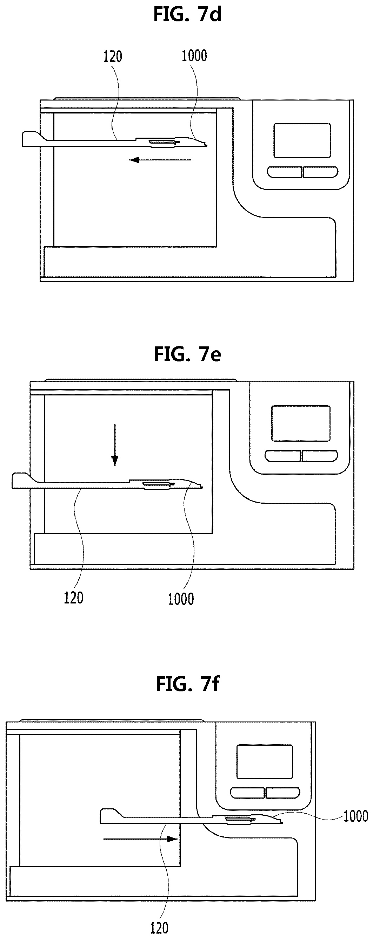

Then, as shown in FIG. 7D, after the seat 120 with the oral sensor 1000 loaded thereon exits the sterilization zone by being moved horizontally by the mover 400, as shown in FIG. 7E, the seat 120 with the oral sensor 1000 loaded thereon reaches to a height where the seat can enter the packaging zone by being moved down by the mover 400, and after that, as shown in FIG. 7F, the seat 120 with the oral sensor 1000 loaded thereon enters the packaging zone by being moved horizontally by the mover 400 (S600).

Here, the seat 120 with the oral sensor 1000 loaded thereon enters the packaging zone by being moved horizontally by the mover 400, and at the same time, the sucker 321 of the opener 320 sucks the wrapping paper by being moved down, and then the sucker 321 of the opener 320 is moved up to open the mouth of the wrapping paper 2000, and thereby a front end of the oral sensor 1000 enters a rear end of the wrapping paper 2000, so the wrapping paper 2000 closely fits the oral sensor 1000 and packaging is completed (S700). Here, the packaging unit 300 may keep the mouth of uppermost wrapping paper 2000 open in advance regardless of the seat 120 with the oral sensor 1000 loaded thereon entering the packaging zone.

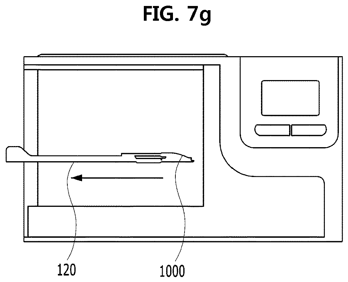

Then, the oral sensor 1000 having been wrapped, as shown in FIG. 7G, can exit the packaging zone by being moved horizontally by the mover 400, wherein as shown in FIG. 7A, the oral sensor may be moved to the outside and wait, or as shown in FIG. 7G, the oral sensor may go out of the packaging zone and wait, but is not limited thereto (S800). Here, before the oral sensor 1000 having been wrapped exits the packaging zone, the oral sensor is moved up to a predetermined height by the mover 400, along with the seat 120, whereby the mover 400 may separate the wrapping paper from the wrapping paper stacker 310, or the oral sensor 1000 having been wrapped may exit the packaging zone in a state where the oral sensor, along with the seat 120, is moved up to a predetermined height by the mover 400.

In performing the above described operation, a display unit 620, such as a light emitting diode (LED), may be provided outside the system to indicate each operation. In other words, of the above described operations, when the sterilization operation (S500) is being performed, the controller 500 may display a guide message "sterilizing" on the display unit 620; and when the sterilization operation (S500) is completed, a guide message "sterilizing completed" may be displayed.

Meanwhile, in order to transmit data generated from the system, a universal serial bus (USB) terminal (not shown) may be provided outside the system, but is not limited thereto.

It is understood by those skilled in the art that the foregoing description is a preferred embodiment of the disclosed device and that various changes and modifications may be made in the invention without departing from the spirit and scope thereof.

## Claims

1. An automatic oral sensor sterilizing and packaging system comprising:
an oral sensor loader configured to provide a seat allowing an oral sensor to be loaded thereon, and to generate a loading signal when the oral sensor is loaded on the seat;
a sterilizer configured to sterilize the oral sensor as the seat enters a sterilization zone;
a packaging unit configured to package the oral sensor with a wrapping paper as the seat enters a packaging zone;
a mover configured to transfer the seat to the sterilization zone in response to input of a first transfer signal, and transfer the seat to the packaging zone in response to input of a second transfer signal; and
a controller configured to generate the first transfer signal in response to input of the loading signal, and generate the second transfer signal as the sterilizer completes sterilization.

2. The system of claim 1, further comprising:
a housing configured to accommodate the oral sensor loader, the sterilizer, the packaging unit, the mover, and the controller therein; and
a door configured to expose the seat to an outside by being openably provided at a side of the housing.

3. The system of claim 2, further comprising:
a motion recognition sensor provided at a side of the housing, and configured to generate a motion recognition signal in response to recognition of motion of the housing, wherein the door is opened in response to input of the motion recognition signal.

4. The system of claim 2, further comprising:
a bed provided at a lower portion of the seat and configured to cover an inside of the housing when the door is opened.

5. The system of claim 1, wherein the seat includes:
a first part configured to allow a sensor part of the oral sensor to be loaded thereon;
a second part configured to allow a cable of the oral sensor to be loaded thereon; and
an anti-twist part provided in the second part, and configured to guide the cable while preventing the cable from being twisted.

6. The system of claim 1, wherein the sterilizer includes:
a light source configured to emit light for sterilization inside the sterilization zone; and
a reflector configured to reflect the light for sterilization inside the sterilization zone.

7. The system of claim 1, wherein
the wrapping paper is in an envelope shape with a mouth thereof being open, and
the packaging unit includes: a wrapping paper stacker configured to allow the wrapping paper to be stacked therein; and an opener configured to open the mouth of a wrapping paper in the packaging zone, of stacked wrapping paper.

8. The system of claim 7, wherein
the opener is provided with a sucker configured to suck a surface of the wrapping paper and move up and down, such that the sucker is moved down to suck the surface of the wrapping paper, and then moves up to open the mouth.

9. The system of claim 1, wherein
the mover is configured to move the seat vertically and horizontally, and
the sterilizer and the packaging unit are disposed on top of the other at a side of a vertical path of the seat.

10. An automatic oral sensor sterilizing and packaging system comprising:
a housing provided with at least one of a sterilizer providing a sterilization zone to sterilize an oral sensor, and a packaging unit providing a packaging zone to package the oral sensor; and
a mover configured to pass the oral sensor through at least one of the sterilization zone and the packaging zone.

11. The system of claim 10, wherein
the sterilizer is configured to either emit light for sterilization or spray sterilizing liquid to the sterilization zone.

12. The system of claim 10, wherein the sterilizer includes:
a light source configured to emit light for sterilization to the sterilization zone; and
a reflector configured to reflect the light for sterilization to the sterilization zone.

13. The system of claim 10, wherein the packaging unit includes:
a wrapping paper supplier configured to supply wrapping paper that is in an envelope shape with a mouth thereof being open, to the packaging zone; and
an opener configured to open the mouth of the wrapping paper.

14. The system of claim 13, wherein the opener includes:
a sucker configured to suck an outer surface of the wrapping paper, and to be movable in an opening direction of the mouth, wherein the sucker opens the mouth by sucking the outer surface of the wrapping paper and by moving in the opening direction.

15. The system of claim 13, wherein the mover includes a seat allowing the oral sensor to be loaded thereon, and is configured to pass the seat through at least one of the sterilization zone and the packaging zone.

16. The system of claim 15, wherein the sterilization zone and the packaging zone are disposed along a first direction, and
the mover is configured to move the seat in the first direction, and in a second direction intersecting with the first direction.

17. The system of claim 15, further comprising:
a door openably provided at a side of the housing, such that the oral sensor is loaded on the seat through the door.

18. The system of claim 17, further comprising:
a motion recognition sensor provided at a side of the housing, and configured to open and close the door in response to a user's motion.
